Europäisches Patentamt

(19) European Patent Office

Office européen des brevets

(11) Publication number: 0 300 059

A1

## (12) EUROPEAN PATENT APPLICATION

published in accordance with Art. 158(3) EPC

(21) Application number: 88901458.5

(22) Date of filing: 04.02.88

Data of the international application taken as a basis:

(86) International application number:
PCT/JP88/00107

(87) International publication number:
WO88/05957 (11.08.88 88/18)

(51) Int. Cl.³: **G 11 C 17/00**

(30) Priority: 06.02.87 JP 26868/87
19.03.87 JP 67537/87

(43) Date of publication of application:
25.01.89 Bulletin 89/4

(84) Designated Contracting States:
DE FR GB

(71) Applicant: TAKASAGO ELECTRIC INDUSTRY CO. LTD.
9-10, Imazukita 4-chome Tsurumi-ku
Osaka-shi Osaka 538(JP)

(72) Inventor: HAMANO, Junichi
24-13, Koshienguchikitamachi
Nishinomiya-shi Hyogo 663(JP)

(74) Representative: Morgan, James Garnet et al,
MANITZ FINSTERWALD & ROTERMUND
Robert-Koch-Strasse 1
D-8000 München 22(DE)

(54) ELECTRONIC COMPONENT PROCESSING APPARATUS.

(57) Electronic component processing apparatus for executing consecutively processing such as data writing to electronic components. The apparatus comprises a conveyor unit for conveying electronic components as the object of processing, a supply unit for supporting trays, in which a large number of electronic components are arranged and stored and which are disposed upstream of the conveyor unit, continuously with the conveyor unit, a processing unit for executing predetermined data processing on the electronic components supplied from the trays, which is disposed in the conveyor unit, a display unit for effecting predetermined display for the electronic components, which is disposed downstream or upstream of the processing unit, and a collection unit for accepting the processed electronic components while supporting the trays continuously with the conveyor unit, which is disposed downstream of the conveyor unit. The apparatus of the present invention eliminates the troublesome tasks of fitting and removing the electronic component to and from a socket and sticking manually a label to the processed electronic component which have been necessary with conventional P-ROM writers or the like, and can automatically process the electronic components continuously and efficiently.

../..

FIG.1

1. TITLE OF THE INVENTION

Electronic Parts Processing Machine

3. DETAILED DESCRIPTION OF THE INVENTION

<Applicable Industrial Field>

The present invention relates to an electronic parts processing machine for continuously writing or reading programs or data (hereinafter referred to as "information") to or from the electronic parts such as P-ROMs or EP-ROMs.

<Prior Art>

As the electronic parts processing machine of this type, a P-ROM writer may be given.

The P-ROM writer is comprised of a keyboard, an operation made display lamp and a digital display provided on a front panel, on which one or more sockets are disposed. In writing information, at first an electronic parts to be modeled is inserted into the socket to set it in the P-ROM writer as model information, then unprocessed electronic parts are inserted into the socket for writing processing.

<Problems to be solved by the Invention>

In the case of P-ROM writer, however, since the number of processings which can be processed at the same time is equal to the number of sockets disposed on the front panel, it is not only unsuitable for mass processing but also requires much troubles in attaching and detaching the parts to and from the sockets or labeling processed parts, thereby causing problems including cost rise. It is, therefore, an object of the present invention to provide a novel electronic parts processing machine which is able to perform processings such as writing and reading automatically and to solve prior art problems, by disposing a tray in which a number of electronic parts are aligned upstream to an electronic parts transfer path, whereon the electronic parts carried out intermittently are stopped temporarily, and performing processings such as writing and reading to and from the electronic parts and predetermined indication for the electronic parts on the transfer path.

<Means for solving Problems>

In explaining means of the present invention for achieving the aforesaid object, the electronic parts processing machine of the present invention implements such processings as writing and reading of information continuously to and from the electronic parts, and comprises a carrying portion which transfers the electronic parts, a processing parts supplying portion which is disposed continuously upstream to the carrying portion and connects a tray containing a number of parts in line to the carrying portion and supports the tray, a processing portion which is provided on the carrying portion and performs information processing for the electronic parts supplied from the tray, and indicating portion which makes a predetermined indication for the electronic parts located downstream or upstream to the processing portion, and a processed parts collecting portion which is disposed downstream to the carrying portion and connects the tray to the carrying portion and supports the tray to receive processed electronic parts therein.

<Operation >

Electronic parts in a tray supported by the processing parts supplying portion are carried out one by one in response to the processing speed and pass through the transfer path. The parts supplied from the tray stop at the processing portion to receive a predetermined processing such as writing, then further stop at the indicating portion to get a predetermined indication on its surface. Thereafter, the parts move again into the tray disposed on the processed parts collecting portion one after the other.

<Embodiments>

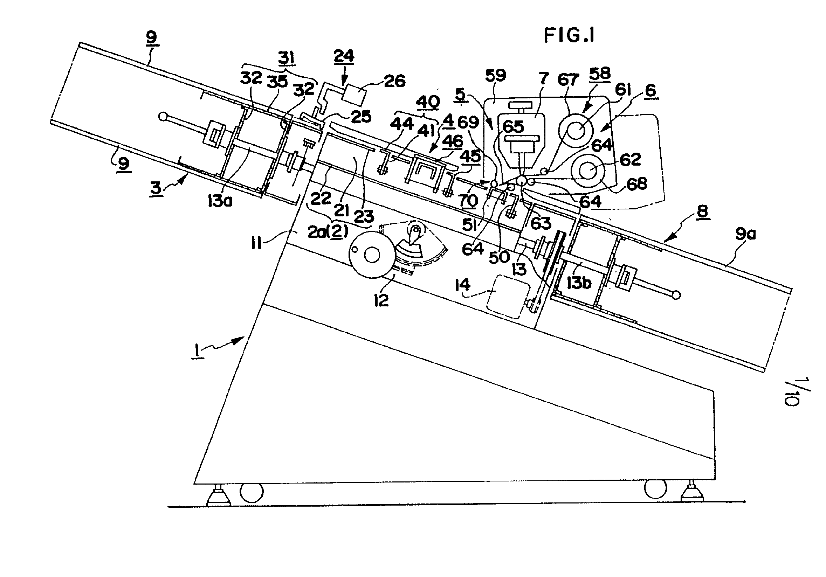

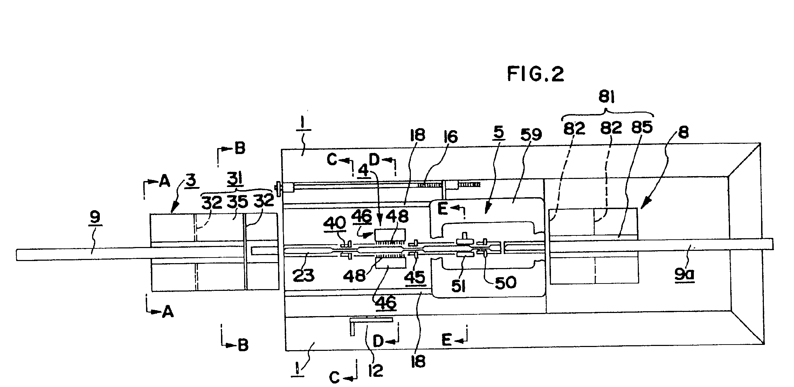

The drawings show an electronic parts processing machine according to one embodiment of the present invention, in which Fig. 1 is a front view and Fig. 2 is a plan view.

The illustrated electronic parts processing machine is an automatic processing machine for continuously performing writing of programs into the electronic parts such as P-ROMs or EP-ROMs, but the present invention is not limited thereto and can be applied to such a machine which performs continuous reading of information from the electronic parts of this type, and further applied to such a machine which performs programming to build hard logic in the electronic parts beside the P-ROMs or EP-ROMs, for example, a PAL (Programmable Array Logic).

The illustrated machine comprises a carrying portion 2 which is located at an upper portion of the machine table 1 and intermittently transfers the electronic parts, a supply portion 3 which is disposed in adjacent and upstream to the carrying portion 2 and has a tray 9 containing many electronic parts in line, a processing portion 4 which is provided in the carrying portion 2 and implements writing of predetermined information into the parts supplied from the tray 9, an indicating portion 5 which is located downstream to the processing portion 4 and makes a predetermined indication for the processed parts, a collecting portion 8 which is disposed downstream to the carrying portion 2 and connects a tubular tray 9a to the carrying portion 2 and supports the tray to collect processed parts, and a control portion (not shown) which consecutively controls an operation of each mechanism and data input/output operations.

On the upper portion of the machine table 1, a mounting frame 11 is provided, which is so made that its angle of inclination can be freely adjusted by an angle adjusting mechanism 12.

The carrying portion 2 is constructed by mounting an inclination guide 2a on the mounting frame 11.

The inclination guide 2a is molded from a high lubricative synthetic resin material. A mounting wall 22 fixed to the frame 11 is formed beneath a supporting wall 21 having a suitable height, and at the upper portion of the supporting wall 21, a parts guide face 23 is formed for engagement with gaps between electrodes of the electronic parts, which is chuted along the inclination.

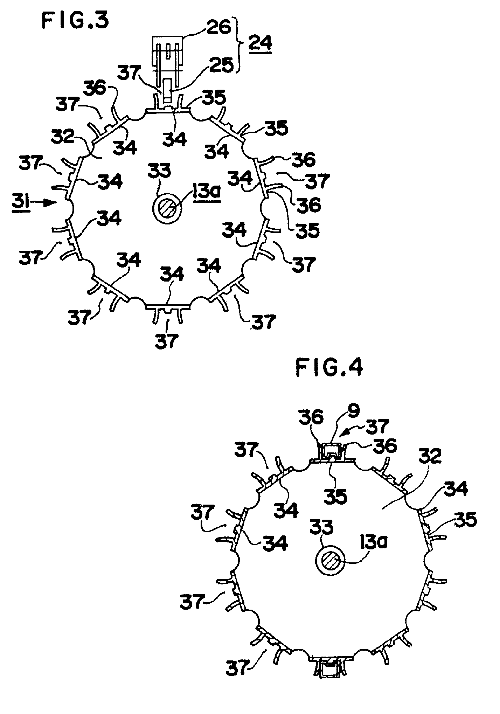

The processing parts supplying portion 3 is constructed by disposing a rotating drive shaft 13 in the mounting frame 11 along the inclination, coupling a stepping motor 14 to the driving shaft 13 whose both ends are projected outside the frame 11, and fixing a tray supporting frame 31 removably to a projected shaft portion 13a on the upstream side. As shown in Figs. 1 - 3, the tray supporting frame 31 is equipped with two opposite disk frame plates 32 each of which is provided respectively with a bearing member 33 at its center and a plurality of attaching sides 34 that are formed on the periphery of the disk frame plate 32 with equal spacings, and tray supporting plates 35 disposed cylindrically between the opposite attaching sides 34 of both disk frame plates 32. Each supporting plate 35 is formed with a tray engaging groove 37 by parallel rising walls 36, and a tubular tray 9 is mounted removably to the tray engaging groove 37.

The tray supporting frame 31 is rotated intermittently by a spacing between the trays disposed cylindrically by the stepping motor 14, and each tray is stopped at a point on an extension of the inclination guide by a position sensor.

Between the inclination guide 2a and the tray supporting frame 31, an open-close mechanism 24 for making the electronic parts in the tray escape one by one is provided.

The open-close mechanism 24 is comprised of a swinging type open-close lever 25 which is disposed opposite to an opening of the tubular tray 9 and a solenoid 26 which swings the lever 25, thus the electronic parts in the tray 9 are carried out one by one into the carrying portion 2 by swinging action of the lever 25. On the upstream side of the carrying portion 2, a stopper 40 is provided to standly one electronic parts supplied from the tray 9 on an upstream side of the processing portion 4.

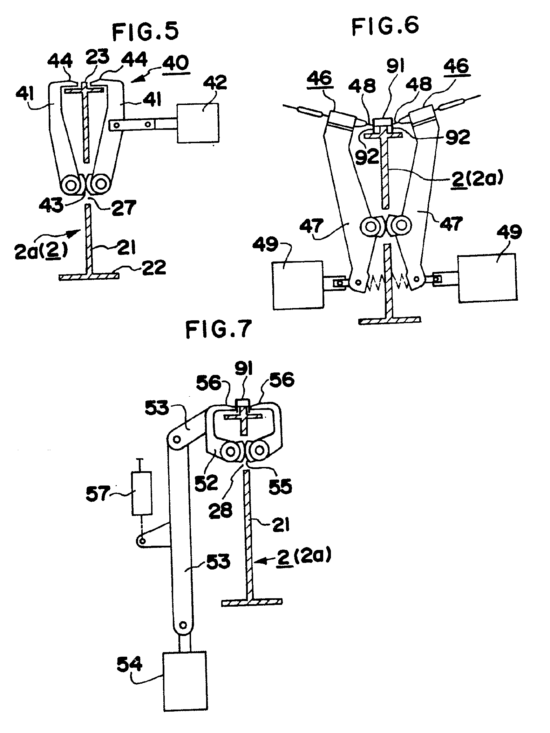

As shown in Fig. 5, the stopper 40 is comprised of a pair of swinging arms 41 which appear and disappear in a parts path in the inclination guide to block their passage, and a solenoid 42 which actuates the swinging arms 41. Both arms 41 are disposed opposite to each other on both sides of the inclination guide 2a and pivotally connected at the lower portions. Beneath the arms 41, gears 43 are formed in one body to engage with each other at a penetrated hole 27. One arm 41 is linked to the solenoid 42 and at the upper ends of both arms 41, stopping pieces 44 which appear and disappear on the parts guide face 23 are formed. By operation of the stopper 40, the electronic parts from the supplying portion 3 are transferred one by one to the processing portion 4.

The processing portion 4 is comprised of a stopper 45 which stops the electronic parts at a predetermined position and a writing portion 46 which writes predetermined information to the parts stopped by the stopper 45.

The stopper 45 has the same structure as the above

0300059

stopper 40 on the upstream side, so its explanation will be omitted.

As shown in Figs. 1 and 6, the writing portion 46 includes writing terminals 48 connected to the control portion on the upper end of a pair of arms 47 which are disposed opposite to each other on both sides of the inclination guide 2a, and solenoids 49 are linked to the lower portions of the arms 47, which are operated by the solenoids 49 to contact the writing terminals 48 to electrodes of the corresponding electronic parts and to write predetermined information by an electric signal from the control portion.

On the downstream side of the processing portion 4, the indicating portion 5 is disposed and a label written in with characters and symbols indicative of written information is affixed to the surface of the processed parts. The indicating portion 5 may be disposed upstream to the processing portion 4. The indicating portion 5 illustrated is comprised of a stopper 50 which stops the processed parts at a predetermined position, a centering mechanism 51 which decides centering of the electronic parts stopped by the stopper 50 and a labeler 58 which affixes a label onto the surface of the electronic parts.

The stopper 50 has the same structure as the stoppers 40, 45, so the explanation will be omitted.

As shown in Fig. 7, the centering mechanism 51 is comprised of a pair of centering arms 52 which are disposed on both sides of the inclination guide, and a solenoid 54 which is connected to one arm 52 via a link 53. On the pivotally connected portion of both arms 52, gears 55 are provided to engage with each other at a position of a penetrated hole 28 in the supporting wall 21, and at an upper portion, nail pieces 56 are formed to grip the electronic parts from both sides. On the link 53 connecting the solenoid 54 and arm 52, a dash-pot 57 is attached to adjust the grip force of the nail pieces 56 which is produced by the solenoid 54.

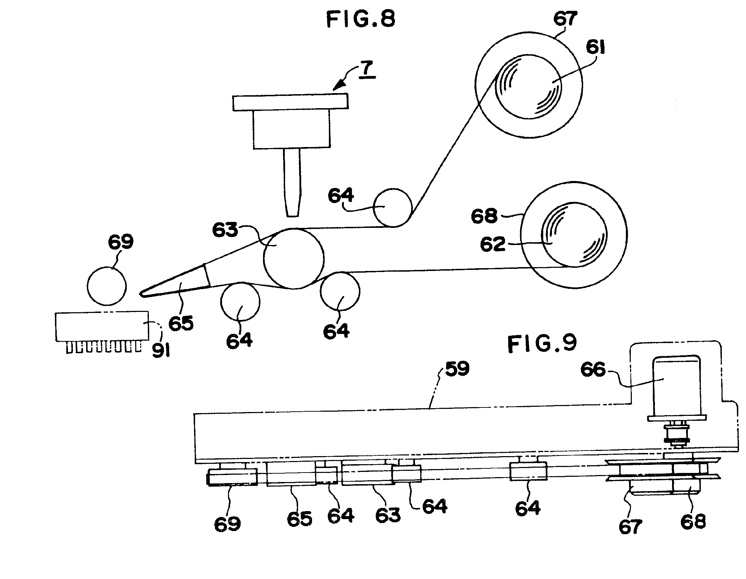

As shown in Figs. 1 - 2, 8 and 9, the labeler 58 is equipped with a ball screw 16 connected to a rotation-reversible drive motor 17 and guide axes 18 in parallel to the inclination guide 2a on the machine table 1, the ball screw 16 and guide axes 18 being provided with a reciprocable table 59, to which a labeling unit 6, printer 7 and press roller 69 are mounted to constitute a labeling mechanism 70 by reciprocating motion of the press roller 69 and table 59.

The aforesaid labeling unit 6 is comprised of reel spindles 61, 62 disposed at the upper and lower portions of the table 59, a supporting roller 63 which is disposed corresponding to the printer, tension rollers 64 which are located respectively on lower both sides of the supporting roller 63 and between the supporting roller 63 and the upper reel spindle 61, and a label peel-off guide 65 which is disposed downstream to the press roller. A stepping motor 66 which operates intermittently is linked to the lower reel spindle 62.

Labels applied to the labeling unit 6 are sticked, in a large number, to a long-rolled paraffin paper tape or the like with equal spacings, which is rolled onto a reel 67 and fit to the upper reel spindle 61. The tape drawn out from the reel 67 passes the tension roller 64 and the upper periphery of the supporting roller 63, turns at the peel-off guide 65, then passes the tension roller 64 and the lower periphery of the supporting roller 63 and is guided by a take-up reel 68. The tape is transferred toward the take-up reel by a label spacing by the motion of the stepping motor 66 to stop the label thereon at an upper surface of the supporting roller 63.

The printer 7 is disposed opposite to the upper periphery of the supporting roller 63 and is actuated in response to a control signal transmitted from the control portion to print data on the label.

The labeling mechanism 70 aligns the label peel-off guide 65 with the stop position of the processed electronic parts, and attains the labeling operation by equally transferring the tape and table 59. When the tape turns at the peel-off guide 65, the label on the tape is pushed out from the tip of the peel-off guide 65, and simultaneously, the table 59 is moved to leave the label on the surface of the corresponding parts so as to be sticked thereto in one body by pressing action of the press roller 69.

The collecting portion 8 of the processed parts is constituted by, in the same way as the supplying portion 3, projecting the drive shaft 13 in the mounting frame 11 therefrom, and fixing a tray supporting frame 81 removable to a projected shaft portion 13b.

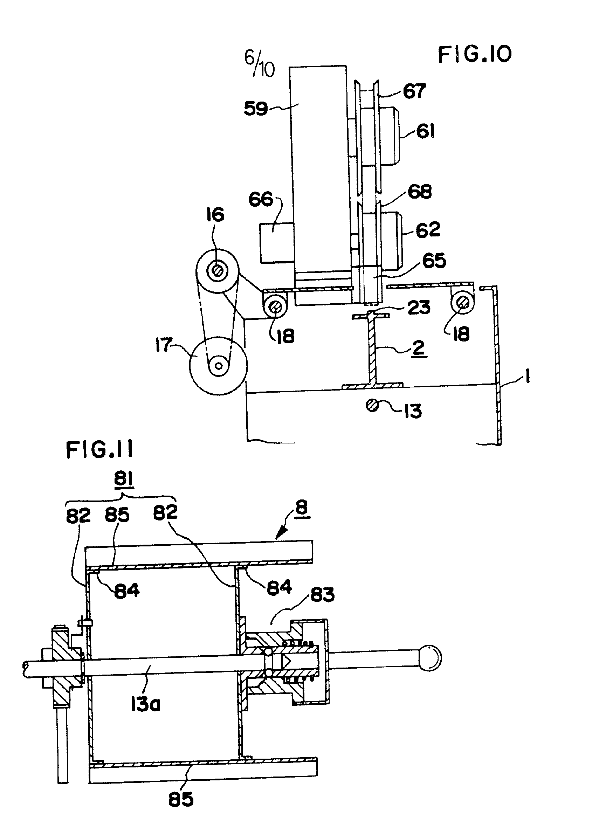

As shown in Fig. 10 and similarly to the supplying side, the tray supporting frame 81 is comprised of two opposite dish frame plates 82 each of which is respectively provided with a bearing member 83 at its center and attaching sides 84 formed on its periphery, and tray supporting plates 85 disposed cylindrically between each attaching side 84 of both disk frame plates 82.

The control portion (not shown) which controls operation of the mechanism or data input/output operations includes a CPU as a control main, ROMs storing programs for such as automatic writing of information, RAMs used to read/write various data, a key operating portion for data input, and a display portion for displaying input/output data.

Information of the writing portion 46 in the mechanism and print data of the printer 7 are previously given to the control portion by operating the key in the control portion.

The indicating portion 5 is not limited to the type of this embodiment, but a type which stamps given characters on a label may be used, or a type which prints or stamps directly on the electronic parts surface may also be used.

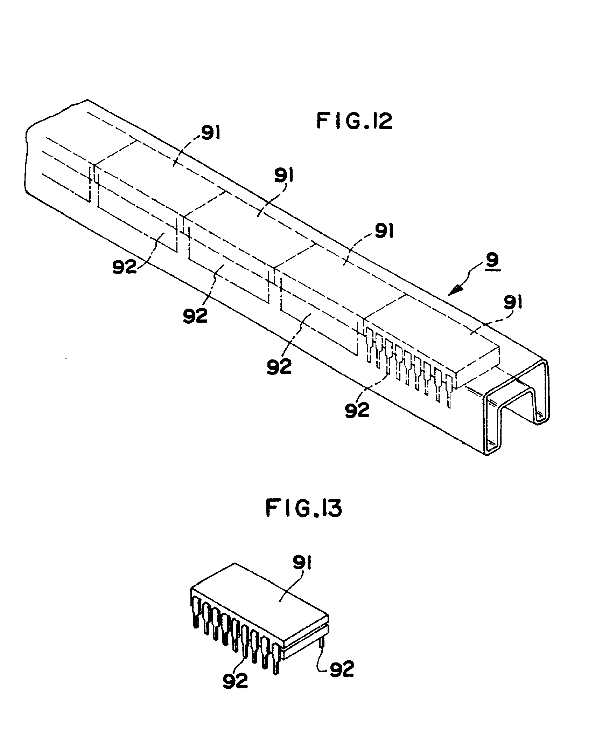

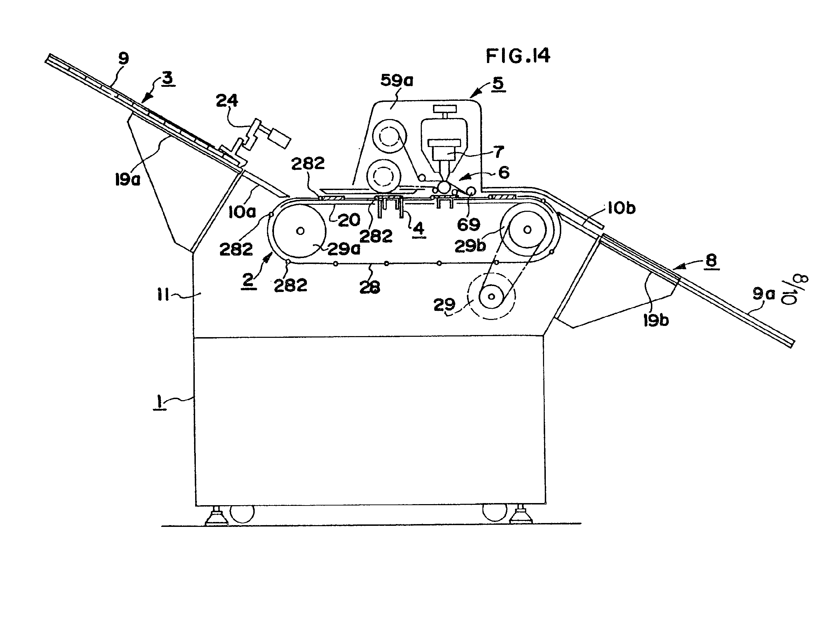

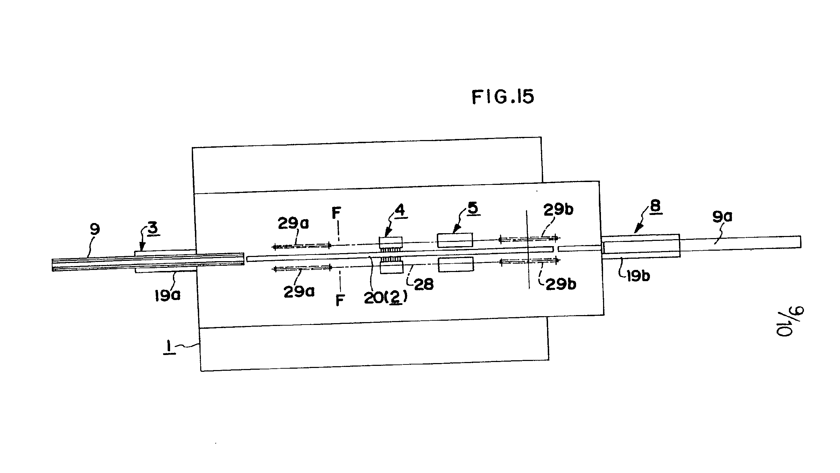

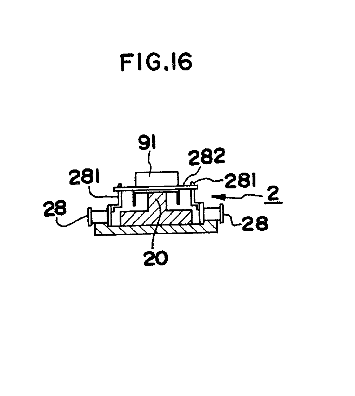

Figs. 13 - 15 show the other embodiment of the electronic parts processing machine of the present invention. This embodiment is so constructed that the parts conveying portion 2 is horizontally disposed, and is comprised of a horizontal carrying guide 20 which performs sliding guide of the electronic parts, and carrying chains 28 which are installed along the guide 20 and force the electronic parts thereon to move forward.

The carrying chains 28 comprises two lines of endless chain stretched between two wheels 29a and 29b oppositely disposed, the former being double-structure driven wheels 29a disposed under the upstream end of the carrying guide 20, and the latter being double-structure drive wheels 29b coupled to a drive motor 29 and disposed under the downstream end. The upper chains are engaged to guide portions on both sides of the carrying guide 20, and between the two lines of endless chains, attaching pieces 281 are projected oppositely in equal spacings. Between the attaching pieces 281, a push rod 282 which moves on the guide 20 is provided to push the back of the electronic parts on the guide 20 to move forward when moving.   In the carrying portion 2,   as same as the previous embodiment, the parts supply portion 3 and collecting portion 8 are provided on the supply and discharge sides,   and in the carrying path 2,   the processing portion 4 which performs information processing into the electronic parts and the indication portion 5 which makes a predetermined indication onto the processed electronic parts are disposed.

The supply portion 3 is provided with an inclined tray mounting frame 19a which has an open-close mechanism 24 at the end of the mounting frame 11 as same as the aforesaid embodiment,   and the tubular tray 9 is mounted removably to the frame 19a.   Between the mounting frame 19a and the carrying portion 2,   a chute is disposed to carry the electronic parts from the tray 19a to the carrying guide 20 therethrough.   The processing portion 4 has the same structure as the previous embodiment, so that the explanation thereof will be omitted.  The labeling unit 6 and the printer 7 which constitute the indicating portion 5 are provided on a fixed frame 59a disposed on the machine table 1.   Thus,   in this embodiment,   a label peeled off by the peel-off guide 65 is transferred to the upper surface of the electronic parts being moved and affixed thereto by passing beneath the press roller 69.   It will be appreciated that,   as same as the previous embodiment,   a movable labeling unit 6 and printer 7 may be introduced in this embodiment.

The parts collecting portion 8 is equipped with a tray mounting frame 19b inclined toward the downstream side at the end of the machine table 1, and an empty tubular tray 9a is installed removably to the frame 19b. Between the frame 19b and carrying portion 2, a chute is provided to carry the processed parts from the carrying portion to the tray 9a therethrough.

It is also possible, as same as the previous embodiment, to constitute the present embodiment by the tray supporting frame 31 provided with a plurality of tubular trays 9 cylindrically.

Now, in the parts processing machine of the first embodiment, when writing information into the electronic parts, the tubular tray 9 containing a number of electronic parts 91 as shown in Fig. 11 is provided on the tray supporting frame 31 on the supply side, and the empty tubular tray 9a on the tray supporting frame 81 on the collecting side.

While, the key in the control portion is operated to supply operation contents of the writing portion 46 and printer 7 to the control portion. At this point of time, each stopper 45, 50 of the inclination guide 2a is respectively positioned in the parts path in the parts guide, thus the electronic parts 91 from the tray 9 is stopped at the stopper 40 on the upstream side of the inclination guide 2a and standing by in the chute. When the stopper 40 performs opening motion, the electronic parts 91 which is standing by is chuted along the parts guide face 23 of the inclination guide 2a and stopped at the processing position by the stopper 45 of the parts processing portion 4. By interlocking motion of the arms 47 in the processing portion 4, the writing portions 46 project each other to electrically connect the writing terminals 48 to electrodes 92 of the corresponding electronic parts 91 and to execute writing of information in response to a control signal from the control portion. At this point, the next electronic parts 91 is standing by at the stopper 40 on the upstream side.

When writing is finished, both arms 47 are operated to detach the writing portion 46 from the electronic parts 91, and the interlocking opening motion of the stopper 45 allows chuting of the electronic parts 91. When the electronic parts 91 is stopped by the stopper 50 of the indicating portion 5, interlocking motions of the stepping motors 66, 17 of the reels 67, 68 and ball screw 16 take place. At this time, the label on the peel-off guide 65 which is printed already extends from the peel-off guide 65 by movements of the tape and table 59, and is remained on the corresponding electronic parts 91 and affixed thereto by pressing action of the corresponding roller 69. At this point of time, the next electronic parts 91 is carried into the processing portion 4 and writing processing is executed in the same way as previously described.

The table 59 reciprocates in accordance with a length of the label, and interlocking to return of the table 59, the stopper 50 is open, thereby carrying the electronic parts 91 to the tubular tray 81 communicating with the guide so as to be collected.

In processing the electronic parts by the machine shown in Figs. 13 - 15, the tubular tray 9 containing the electronic parts is mounted to the mounting frame 19a on the supply side, and the empty tubular tray 9a to the frame 19b on the collecting side.

While, the key in the control portion is operated to supply operation contents of the writing portion 46 and printer 7 to the control portion. When the open-close mechanism 24 of the supplying portion 3 performs open-close operation, one parts in the tray 9 is chuted from the chute onto the guide 20 of the carrying portion 2. Interlocking to this action, carrying chains 28 operate to transfer the parts on the guide 20 to the processing portion. Interlocking thereto, the writing portion 46 operates to write information onto the corresponding electronic parts. Interlocking to completion of writing, the carrying chains 28 operate again to transfer the processed parts to the position of the indicating portion 6 and labeling the surface of the electronic parts is performed by the printer and the labeling unit. Interlocking to completion of labeling, the carrying chains 28 operate again to transfer the processed parts from the chute to the empty tray.

<Advantage of the Invention>

As described hereinabove, the present invention is a machine which implements writing and reading of information for electronic parts continuously, and comprises a carrying portion which transfers the electronic parts, a processing parts supplying portion which is disposed in adjacent and upstream to the carrying portion and having a tray containing a number of electronic parts in line, a processing portion which is provided on the carrying portion and executes predetermined processing for the electronic parts supplied from the tray, an indicating portion which is located downstream or upstream to the processing portion and performs a predetermined indication for the processed electronic parts, and a collecting portion which is disposed downstream to the carrying portion and supports the tray in adjacent to the carrying portion to receive the processed electronic parts, so that the parts in the tray supported by the parts supplying portion are carried out one by one in response to the processing speed, moving the carrying portion and stop at the processing portion, and after predetermined processing stop again at the indicating portion, where a predetermined indication is performed on the parts, and there after move again into the tray disposed in the collecting portion one after the other.

Thus, according to the present invention, troublesome works such as taking out the electronic parts from the tray, attaching or detaching it to and from sockets of the P-ROM writer and inserting again into the tray as in the conventional P-ROM writer can be eliminated, thereby achieving the object of the invention such as enabling automatic and continuous processing of the electronic parts.

4. BRIEF DESCRIPTION OF THE DRAWINGS

Fig. 1 is a front view of an electronic parts processing machine according to one embodiment of the present invention,

Fig. 2 is a plan view,

Fig. 3 is a sectional view taken along the line A-A of Fig. 2,

Fig. 4 is a sectional view taken along the line B-B of Fig. 2,

Fig. 5 is a sectional view taken along the line C-C of Fig. 2,

Fig. 6 is a sectional view taken along the line D-D of Fig. 2,

Fig. 7 is a sectional view taken along the line E-E of Fig. 2,

0300059

Figs. 8 and 9 are views showing one example of a labeling unit,

Fig. 10 is a sectional view showing a parts collecting portion,

Fig. 11 is a perspective view of a tubular tray,

Fig. 12 is a perspective view showing one example of an electronic parts,

Fig. 13 is a front view showing the other embodiment of an electronic parts processing machine,

Fig. 14 is a plan view of Fig. 13, and

Fig. 15 is a sectional view taken along the line F-F of Fig. 14.

| | | | |
|---|---|---|---|
| 2 | .... carrying portion | 3 | .... supplying portion |
| 4 | .... processing portion | 8 | .... collecting portion |
| 9 | .... tray | 91 | .... electronic parts |

Applicant: Takasago Electric Industry Co., Ltd.

Agent: Yoshimitsu SUZUKI, Patent Attorney

2. WHAT IS CLAIMED IS:

(1) An electronic parts processing machine for continuously performing processes like writing or reading information to or from the electronic parts, comprising:

a carrying portion for transferring the electronic parts;

a supplying portion of parts to be processed which is disposed upstream to the carrying portion and connects a tray containing a number of electronic parts in line to the carrying portion and supports the tray;

a processing portion which is disposed in said carrying portion and performs information processing for the electronic parts supplied from said tray;

an indicating portion for making a predetermined indication for the electronic parts and located downstream or upstream to said processing portion; and

a collecting portion for processed parts which is disposed downstream to the carrying portion and connects the tray to the carrying portion and supports the tray to receive the processed electronic parts.

(2) An electronic parts processing machine according to claim 1, wherein said carrying portion is constituted by arranging stoppers suitably along an inclination guide whereon the electronic parts are chuted by gravity.

(3) An electronic parts processing machine according to claim 1, wherein said carrying portion is comprised of a carrying guide for guiding the electronic parts and carrying chains for transferring the electronic parts placed along the carrying guide.

(4) An electronic parts processing machine according to claim 1, wherein said indicating portion is comprised of a labeling mechanism which affixes the label printed by a printer.

(5) An electronic parts processing machine according to claim 1, wherein said processing parts supplying portion and processed parts collecting portion are provided with a supporting frame which supports a plurality of trays in parallel and cylindrically.

FIG.1

FIG.2

FIG.3

FIG.4

FIG.5

44 23 44 40
41        41    42
43    27
2a(2)    21
22

FIG.6

46  48  91  48  46
92        92
2(2a)
49  47        47  49

FIG.7

91
56    56
53
52    55
57    28
21
2(2a)
53
54

FIG.8

FIG.9

6/10

**FIG.10**

59

67

61

66

68

62

16

65

17

18

23

18

2

1

13

**FIG.11**

81

82 85 82

8

84

84

83

13a

85

FIG.12

91

91

91

92

92

92

92

9

91

91

FIG.13

91

92

92

# FIG.14

FIG.15

FIG.16

# INTERNATIONAL SEARCH REPORT

0300059

International Application No  PCT/JP88/00107

**I. CLASSIFICATION OF SUBJECT MATTER** (if several classification symbols apply, indicate all) [3]

According to International Patent Classification (IPC) or to both National Classification and IPC

Int.Cl$^4$   G11C17/00

**II. FIELDS SEARCHED**

Minimum Documentation Searched [4]

| Classification System | Classification Symbols |
|---|---|
| IPC | G11C17/00 |

Documentation Searched other than Minimum Documentation
to the Extent that such Documents are Included in the Fields Searched [5]

| | | |
|---|---|---|
| Jitsuyo Shinan Koho | 1973 | 1987 |
| Kokai Jitsuyo Shinan Koho | 1974 | 1987 |

**III. DOCUMENTS CONSIDERED TO BE RELEVANT** [14]

| Category * | Citation of Document, [16] with indication, where appropriate, of the relevant passages [17] | Relevant to Claim No. [18] |
|---|---|---|
| X | JP, A, 62-22296 (Mitsubishi Electric Corp.) 30. January. 1987 (30. 01. 87) (Family: none) | 1-5 |
| X | JP, A, 62-3489 (Mitsubishi Electric Corp.) 9. January. 1987 (09. 01. 87) (Family: none) | 1-5 |
| X | JP, A, 61-237297 (USAC Denshi Kogyo Kabushiki Kaisha) 7. October. 1986 (07. 10. 86) (Family: none) | 1-5 |

* Special categories of cited documents: [16]

"A"  document defining the general state of the art which is not considered to be of particular relevance

"E"  earlier document but published on or after the international filing date

"L"  document which may throw doubts on priority claim(s) or which is cited to establish the publication date of another citation or other special reason (as specified)

"O"  document referring to an oral disclosure, use, exhibition or other means

"P"  document published prior to the international filing date but later than the priority date claimed

"T"  later document published after the international filing date or priority date and not in conflict with the application but cited to understand the principle or theory underlying the invention

"X"  document of particular relevance: the claimed invention cannot be considered novel or cannot be considered to involve an inventive step

"Y"  document of particular relevance: the claimed invention cannot be considered to involve an inventive step when the document is combined with one or more other such documents, such combination being obvious to a person skilled in the art

"&"  document member of the same patent family

**IV. CERTIFICATION**

| Date of the Actual Completion of the International Search [3] | Date of Mailing of this International Search Report [3] |
|---|---|
| April 11, 1988 (11. 04. 88) | April 25, 1988 (25. 04. 88) |

| International Searching Authority [1] | Signature of Authorized Officer [20] |
|---|---|
| Japanese Patent Office | |

Form PCT/ISA/210 (second sheet) (October 1977)